# EUROPÄISCHE PATENTSCHRIFT

(11) **EP 0 767 169 B1**
(45) Veröffentlichungstag und Bekanntmachung des Hinweises auf die Patenterteilung: **30.12.1998**
(21) Anmeldenummer: 96115818.5
(22) Anmeldetag: 02.10.1996
(51) Int. Cl.: C07D 207/267

(54) **Verfahren zur Reinigung von N-Vinylpyrrolidon durch Kristallisation**
Process for the purification of N-vinylpyrrolidone by crystallisation
Procédé de purification de N-vinylpyrrolidone par cristallisation

(30) Priorität: 02.10.1995 DE 19536859
(43) Veröffentlichungstag der Anmeldung: 09.04.1997
(73) Patentinhaber: BASF AKTIENGESELLSCHAFT, 67056 Ludwigshafen (DE)
(72) Erfinder: Schmidt-Radde, Martin, Dr., 67259 Beindersheim (DE); Helfert, Herbert, Dr., 67227 Frankenthal (DE); Eck, Bernd, 68519 Viernheim (DE); Maltry, Bernhard, 67283 Obrigheim (DE)
(74) Vertreter: Isenbruck, Günter, Dr.

(56) Entgegenhaltungen:
- DE-A- 3 736 603
- US-A- 5 329 021
- DATABASE WPI Derwent Publications Ltd., London, GB; AN 78-77756a XP002022247 & SU-A-582 247 (HORMONE BLOOD SUBST.) , 25.November 1977

## Beschreibung

Die vorliegende Erfindung betrifft ein Verfahren zur Reinigung von N-Vinylpyrrolidon durch Kristallisation.

Polymerisiertes Vinylpyrrolidon findet in vielfältiger Weise für kosmetische Produkte, im Pharmabereich oder in der Lebensmittelindustrie Anwendung.

Bei der Herstellung von N-Vinylpyrrolidon durch Vinylierung von Pyrrolidon mit Acetylen wird nach Synthese und Destillation ein Produkt erhalten, das 1 bis 10 Gew.-% Pyrrolidon sowie Stickstoff- und Vinyletherverbindungen im ppm-Bereich als Verunreinigungen enthält. Für die oben genannten Anwendungen wird eine Gesamtverunreinigung des Produkts von >0,1 Gew.-% nicht mehr toleriert. Darüber hinaus können einzelne Stickstoffverbindungen zu unzulässigen Produktverfärbungen und zu Problemen bei der Polymerisation führen.

Es ist bekannt, daß die Stickstoff- und Vinyletherverbindungen aus dem verunreinigten N-Vinylpyrrolidonprodukt durch Destillation nur unter erheblichem Aufwand abtrennbar sind. In der Offenlegungsschrift DE 37 36 603 (BASF) wird ein Verfahren offenbart, bei dem die Verunreinigungen mit einem sauren Ionentauscher entfernt werden. In der Patentschrift US 5,329,021 (ISP) ist die Reinigung von N-Vinylpyrrolidon durch mehrstufige fraktionierte Kristallisation beschrieben.

Die Anwendung der fraktionierten Kristallisation, wie sie in US 5,329,021 beschrieben ist, hat jedoch den Nachteil einer relativ schlechten Reinigungswirkung je Kristallisationsstufe. In dem US-Patent wird das zu reinigende Vinylpyrrolidon der ersten Reinigungsstufe zugeführt, in der man es auf eine Temperatur zwischen etwa 1°C und 5°C unterhalb des Schmelzpunktes des zugeführten Stroms abkühlt, wobei eine kristalline N-Vinylpyrrolidonphase und eine flüssige Rückstandsphase entstehen. Anschließend trennt man die beiden Phasen voneinander, erwärmt die kristalline Phase auf eine Temperatur, bei der sich die Kristalle verflüssigen (Schmelze) und unterwirft die Schmelze weiteren Kristallisationsschritten. Nachteilig ist hierbei, daß die Schmelze bei der Kristallisation unterkühlt wird, wodurch es zu unkontrolliertem Kristallwachstum kommt, was die Reinigungswirkung verschlechtert.

Außerdem ist mit der in US 5 329 021 beschriebenen Verschaltung der Abtriebsstufen aus folgenden Gründen eine bestimmte Ausbeute nur mit erhöhtem apparativem Aufwand zu erreichen. Das beschriebene Verfahren beruht auf dem bekannten Gegenstromprinzip, bei dem nach jeder Kristallisationsstufe das Kristallisat vom Kristallisationsrückstand abgetrennt wird und die jeweiligen Kristallisatströme den Stufen mit der nächsthöheren Stufennummer zugeführt werden, während die Kristallisationsrückstandsströme den Stufen mit der nächstniedrigeren Stufennummer zugeführt werden. Dabei unterteilt der eintretende, zu reinigende Strom die Stufen in sog. Reinigungs- und Abtriebsstufen. Da auch mehrstufige Schichtkristallisationsanlagen üblicherweise mit nur einem Kristallisationsapparat betrieben werden, benötigt man Pufferbehälter zwischen den einzelnen Kristallisationsstufen. Das bedeutet, daß der apparative Aufwand des Verfahrens mit der Zahl der erforderlichen Trennstufen zunimmt.

Der Erfindung lag daher die Aufgabe zugrunde, das Verfahren der fraktionierten Kristallisation für die Reinigung von N-Vinylpyrrolidon so zu modifizieren, daß gegenüber den bisher bekannten Verfahren deutliche Vorteile im Hinblick auf den Reinigungsaufwand und damit die Wirtschaftlichkeit der Reinigung erzielt werden.

Überraschenderweise wurde gefunden, daß sich dieses Problem dadurch lösen läßt, indem man vor der Kristallisation auf die Flächen des Kristallisators, von denen aus während der Kristallisation Kristalle wachsen, eine Impfschicht aus N-Vinylpyrrolidon aufbringt.

Somit betrifft die Erfindung ein Verfahren zur Reinigung von N-Vinylpyrrolidon durch Kristallisation in einem Kristallisator, wobei man vor der Kristallisation diejenigen Flächen des Kristallisators, von denen aus während der Kristallisation Kristalle wachsen, mit einer Impfschicht aus N-Vinylpyrrolidon belegt. Bevorzugte Ausführungsformen dieses Verfahrens sind in den Unteransprüchen definiert.

Der erfindungsgemäß verwendete Kristallisator unterliegt an sich keiner besonderen Beschränkung. Als besonders geeignet haben sich Kristallisatoren erwiesen, deren Funktion auf der Bildung von Kristallen auf gekühlten Flächen beruht. Geeignete Apparate sind in DE 26 06 364, DE 17 69 123, EP 0 218 545, EP 0 323 377, CH 645 278, FR 266 89 46 und US 3,597,164 beschrieben.

Erfindungsgemäß wird an diesen Stellen oder Flächen, auf denen sich Kristalle bilden sollen, eine Impfschicht aus N-Vinylpyrrolidon vor der Kristallisation erzeugt. Die erfindungsgemäß geeigneten Verfahren zur Erzeugung der Impfschicht unterliegen an sich keiner Beschränkung. Erfindungsgemäß kann zur Erzeugung der Impfschicht sowohl eine N-Vinylpyrrolidon-Schmelze als auch eine N-Vinylpyrrolidon-Lösung verwendet werden. Wird daher im folgenden der Begriff N-Vinylpyrrolidon-Schmelze oder Schmelze verwendet, so gilt dieser Begriff gleichermaßen für eine N-Vinylpyrrolidon-Lösung bzw. Lösung. In einer bevorzugten Ausführungsform der Erfindung wird diese Impfschicht durch Anfrieren eines N-Vinylpyrrolidon-Schmelzefilms erzeugt, der diese Stellen oder Flächen benetzt. Vorzugsweise wird hierzu eine Unterkühlung an den Kristallisatorflächen herbeigeführt. In einer weiteren bevorzugten Ausführungsform der Erfindung wird diese Impfschicht dadurch erzeugt, daß eine zweiphasige Schicht aus N-Vinylpyrrolidon-Schmelze mit suspendierten N-Vinylpyrrolidon-Kristallen auf die Kristallisatorflächen aufgebracht wird. Dies hat den Vorteil, daß anders als beim Anfrieren der einphasigen, flüssigen Impfschicht weniger stark gekühlt werden muß, da die suspendierten Kristalle bereits als Kristallisationskeime wirken. Vorzugsweise liegt hierbei die Kühltemperatur bei der Gleichgewichtstemperatur (Schmelztemperatur) der verwendeten Suspension.

Die Erzeugung der zweiphasigen Suspension unterliegt an sich keiner Beschränkung. In einer bevorzugten Ausführungsform der Erfindung friert man aus einer Schmelze des zu trennenden Gemisches Kristalle aus und bringt diese in die Schmelze ein. Bevorzugt werden in sogenannten Kratzkühlern oder Rührkesseln insbesondere mit wandgängigen Rührern durch indirekte Kühlung Kristalle ausgefroren, die mit Hilfe von Schabelementen von den gekühlten Wänden in die Suspension gefördert werden. Daneben besteht auch die Möglichkeit, durch Abkühlen der Schmelze entweder über den Kristallisator selbst oder über in den Kristallisator oder andere Apparate eingebaute kühlbare Elemente (z.B. Kühlfinger, Kühlstrecken oder Rührbehälter) Kristalle direkt in der Schmelze oder Lösung zu erzeugen und auf diese Weise eine Suspension zu erzeugen. Dies hat den Vorteil, daß die Kristalle nicht abgeschabt werden müssen. Die Verwendung kühlbarer Elemente ist vorteilhaft, da nicht der gesamte Kristallisator abgekühlt werden muß. Es ist auch möglich, daß man im Kristallisator oder außerhalb von diesem eine Suspension erzeugt und die Kristalle aus der Suspension im Kristallisator auf die Kristallisationsflächen sedimentieren läßt, wo sie als Impfkristalle wirken. Der Feststoffgehalt der Suspension liegt zwischen 0 g Feststoff/g Suspension und 60 g Feststoff/g Suspension.

In einer bevorzugten Ausgestaltung der Erfindung bringt man die Suspension auf die Kristallisatorflächen auf, indem man den Kristallisator mit der Suspension füllt und anschließend entleert. Nach der Entleerung verbleibt eine Suspensionsschicht auf den Kristallisatorflächen, die dann (bei ihrer Gleichgewichtstemperatur) angefroren wird. Entsprechend kann verfahren werden, wenn die Suspension im Kristallisator selbst erzeugt wird. Daneben besteht auch die Möglichkeit, die Suspension über übliche Verteilervorrichtungen (z.B. Düsensysteme oder Platten) auf die Kristallisatorflächen aufzubringen. Dadurch kann ein Befüllen des Apparates mit anschließendem Entleeren vermieden werden.

Als Impfmaterial wird in beiden Ausführungsformen vorzugsweise das die jeweilige Kristallisationsstufe verlassende Kristallisat verwendet. Daneben besteht jedoch auch die Möglichkeit, anderes N-Vinylpyrrolidon mit höherer oder niedrigerer Reinheit, vorzugsweise höherer Reinheit, zu verwenden. Nach der Erzeugung der Impfschicht wird die Kristallisation durchgeführt.

Die Verfahren zur Durchführung der Kristallisation unterliegen an sich keiner besonderen Beschränkung und sind dem Fachmann bekannt. Geeignete Kristallisationsverfahren sind z.B. in US 5,329,021, DE 26 06 364, DE 17 69 123 und EP 475 893 beschrieben. Erfindungsgemäß wird nach Aufbringen der Impfschicht das zu reinigende Vinylpyrrolidon als Schmelze oder Lösung der ersten Reinigungsstufe zugeführt, in der man es auf seine Gleichgewichtstemperatur (Schmelzpunkt) abkühlt, wobei eine kristalline N-Vinylpyrrolidonphase und eine flüssige Rückstandsphase entstehen. Je nach Zusammensetzung des zu trennenden N-Vinylpyrrolidons liegt die Gleichgewichtstemperatur zwischen +14,4 und -6°C. Diese beiden Phasen werden voneinander getrennt. Anschließend erwärmt man die abgetrennte kristalline Phase auf eine Temperatur, bei der sich die Kristalle verflüssigen (Schmelze), und unterwirft diese Schmelze ggf. weiteren Kristallisationsschritten. Ebenso kann die Rückstandsphase weiteren Kristallisationsschritten unterworfen werden, wie es weiter unten näher beschrieben ist.

Das erfindungsgemäße Kristallisationsverfahren wird in einer oder mehreren Kristallisationsstufen durchgeführt. Allgemein kann man die Kristallisationsstufen in Reinigungsstufen und Abtriebsstufen einteilen. In den Reinigungsstufen und Abtriebsstufen wird jeweils eine Kristallisation durchgeführt, wobei in den Reinigungsstufen das zu reinigende N-Vinylpyrrolidon oder ein reineres Kristallisat und in den Abtriebsstufen der weniger reine Kristallisationsrückstand eingesetzt wird. Das zu reinigende N-Vinylpyrrolidon wird der ersten Reinigungsstufe zugeführt und nach Aufbringen einer Impfschicht aus N-Vinylpyrrolidon einer Kristallisation unterworfen. Nach der Kristallisation wird das Kristallisat vom Kristallisationsrückstand abgetrennt und der nächsten Reinigungsstufe zugeführt, in der ebenso verfahren wird. Der Kristallisationsrückstand der ersten Reinigungsstufe wird der Stufe mit der nächstniedrigeren Stufennummer, einer Abtriebsstufe, zugeführt und dort entsprechend einer Kristallisation unterworfen. Dieses Verfahren beruht auf dem bereits in den Druckschriften DE 26 06 364, DE 17 69 123, EP 475 893 und US 5,329,021 beschriebenen Verfahren des Gegenstromprinzips. Hierbei werden die Kristallisatströme den Stufen mit der nächsthöheren Stufennummer und die Kristallisationsrückstandsströme den Stufen mit der nächstniedrigeren Stufennummer zugeführt.

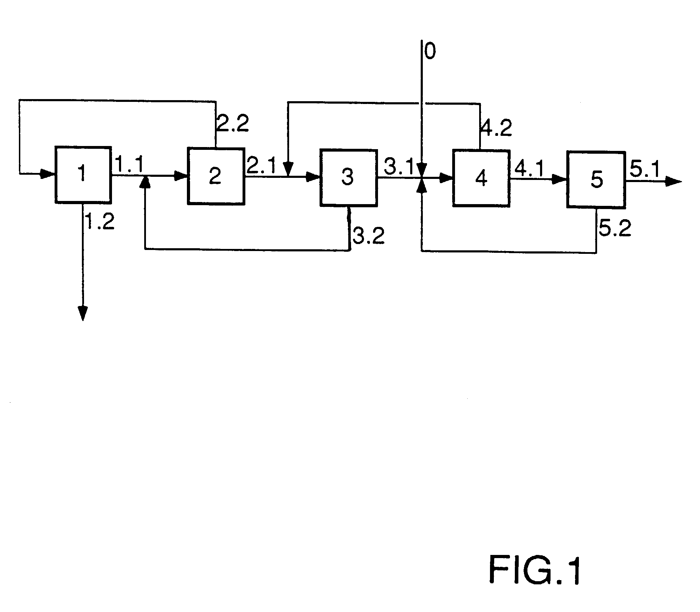

In Fig. 1 ist ein Beispiel einer 5-stufigen Kristallisationsanlage gezeigt, wie sie bei Durchführung des erfindungsgemäßen Verfahrens verwendet werden kann. Hierbei wird der zu reinigende Strom (0) in die Stufe 4 (erste Reinigungsstufe) geleitet und einer Kristallisation unterzogen. Das Kristallisat (4.1) der Stufe 4 wird der folgenden Stufe 5 (zweite Reinigungsstufe) zugeführt und einer Kristallisation unterzogen. Das Kristallisat der Stufe 5 verläßt diese als Strom (5.1), der das gewünschte aufgereinigte N-Vinylpyrrolidon darstellt. Der Kristallisationsrückstand der Stufe 4 wird als Strom 4.2 der Abtriebsstufe 3 zugeführt. Der Kristallisationsrückstand aus der Stufe 5 (Strom 5.2) wird der Stufe 4 zugeführt. Entsprechend wird in den anderen Stufen verfahren, d.h., es werden einer Stufe das Kristallisat aus der vorhergehenden Stufe und der Kristallisationsrückstand aus der nachfolgenden Stufe zugeführt. Hierbei unterteilt der Eintrittsstrom (0) die Stufen in die Reinigungsstufen 4 und 5 und die Abtriebsstufen 1 bis 3, die zusammen den Kristallisationsstufen 1 bis 5 entsprechen. Bei der Durchführung des erfindungsgemäßen Verfahrens wird in jeder Kristallisationsstufe vor der Kristallisation eine Impfschicht aus N-Vinylpyrrolidon auf die Kristallisationsflächen aufgebracht.

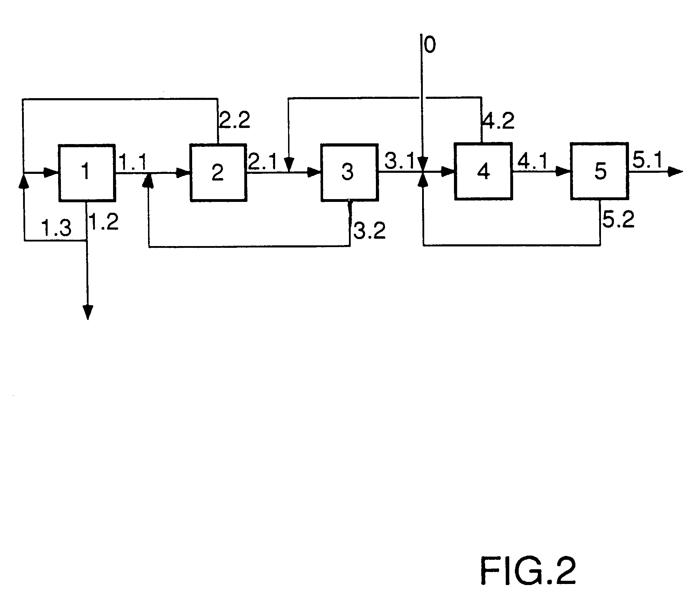

In einer bevorzugten Ausführungsform der Erfindung wird der in der ersten Kristallisationsstufe austretende Kristallisationsrückstand dieser Stufe teilweise wieder rückgeführt. Eine solche bevorzugte Ausführungsform ist in Fig. 2 veranschaulicht. Dabei bezeichnen gleiche Ziffern wie in Fig. 1 gleiche Stufen bzw. Ströme. Der in der ersten Kristallisationsstufe (Abtriebsstufe 1) austretende Kristallisationsrückstand (1.2) wird dieser Stufe teilweise wieder rückgeführt als Strom (1.3.) Das Verhältnis von Strom (1.2) zu Strom (1.3) (Rückführverhältnis) liegt je nach Aufgabenstellung zwischen 0 und 1,0.

Vorzugsweise liegt das Verhältnis von in der ersten Kristallisationsstufe austretendem zu rückgeführtem Kristallisationsrückstand (Rückführverhältnis) zwischen 0,1 und 0,95. Die Anzahl der Kristallisationsstufen und damit auch der Reinigungs- und Abtriebsstufen hängt von der Trennaufgabe ab und kann vom Fachmann im Rahmen üblicher Versuche ermittelt werden. Jedenfalls umfaßt die Kristallisation mindestens eine Kristallisationsstufe, davon mindestens eine Reinigungsstufe. Bevorzugt sind zwei Reinigungsstufen und eine Abtriebsstufe.

Das zu reinigende N-Vinylpyrrolidon unterliegt an sich keiner Beschränkung. Erfindungsgemäß kann jedes beliebige N-Vinylpyrrolidon verwendet werden. Insbesondere wird aus der Vinylierung von Pyrrolidon mit Acetylen hergestelltes N-Vinylpyrrolidon verwendet. Dieses N-Vinylpyrrolidon kann in geeigneter Weise, zum Beispiel durch Destillation, vorgereinigt werden.

Das erfindungsgemäße Verfahren kann als dynamisches oder statisches Verfahren durchgeführt werden oder als Kombination dieser beiden Verfahren. Vorzugsweise finden statische Verfahren im Abtriebsteil der Anlage Anwendung. Bei den statischen Verfahren, wie sie z.B. in US 3 597 164, EP 0 323 377 und FR 2 668 946 beschrieben sind, wird die flüssige Phase nur durch freie Konvektion bewegt (ruhende Schmelze), während bei den dynamischen Verfahren die Kristallisation bei einer erzwungenen Konvektion der flüssigen Phase durchgeführt wird, d.h. die Schmelze ist in strömender Bewegung. Dies kann durch eine erzwungene Strömung in volldurchströmten Wärmeüberträgern erfolgen, wie z.B. in DE 26 06 364 beschrieben, oder durch die Aufgabe eines Rieselfilms auf eine gekühlte Wand erfolgen, wie es z.B. DE-AS 17 69 123 und EP-B 0 218 545 beschreiben.

Nach Durchführung des erfindungsgemäßen Verfahrens können zusätzliche Reinigungsschritte ausgeführt werden. Besonders geeignet sind ein Waschen der Kristallschicht, vorzugsweise mit einer Reinigungsflüssigkeit, z.B. der Restflüssigkeit (Mutterlauge) aus der vorangegangenen Kristallisation, wie in DE 37 08 709 beschrieben, und/oder die Durchführung eines sogenannten Schwitzens der Kristallschicht. Beim Schwitzen wird die Temperatur der Kristallschicht angehoben, wobei bevorzugt die höher verunreinigten Bereiche der Kristallschicht abschmelzen und so eine zusätzliche Reinigungswirkung erzielt wird.

Das nach dem erfindungsgemäßen Verfahren gereinigte N-Vinylpyrrolidon enthält 99,5 bis 99,999 Gew.-% bezogen auf 100 Gew.-% gereinigtes N-Vinylpyrrolidon oder 5000 bis 10 ppm Verunreinigungen. Dieses N-Vinylpyrrolidon genügt den Anforderungen, wie sie im Bereich der Lebensmittelindustrie oder im Pharmabereich gestellt werden.

Die Erfindung wird anhand der folgenden Beispiele, die bevorzugte Ausführungsformen der Erfindung darstellen, näher erläutert.

### Beispiel 1

N-Vinylpyrrolidon mit einer Ausgangsverunreinigung von 0,6 Gew.-% bezogen auf 100 Gew.-% N-Vinylpyrrolidon (diese Verunreinigungskonzentration entspricht der im US-Patent US 5,329,021 genannten Verunreinigungskonzentration) wurde in einem Kristallisator, wie er in der Offenlegungsschrift DE-A-26 06 364 (BASF) beschrieben ist, in zwei Reinigungsstufen und vier Abtriebsstufen kristallisiert. Hierbei wurde in allen Kristallisationsstufen als Impfschicht auf die Flächen des Kristallisators eine zweiphasige Suspension bestehend aus N-Vinylpyrrolidonschmelze mit suspendierten N-Vinylpyrrolidon-Kristallen aufgebracht. Zum Erzeugen der Impfschicht wurde der Kristallisator mit der in der jeweiligen Stufe zu reinigenden Schmelze gefüllt und durch Abkühlen des Apparats eine Suspension erzeugt. Danach wurde der Apparat entleert und die auf den Kristallisatorflächen verbleibende Suspension festgefroren. Die Gleichgewichtstemperaturen der Schmelzen während der Kristallisation in den einzelnen Stufen waren wie folgt:

| | |
|---|---|
| Reinigungsstufe 5 | 13,5 bis 12,9°C |
| Reinigungsstufe 6 | 13,8 bis 13,6°C |
| Abtriebsstufe 1 | 13,1 bis 12,6°C |
| Abtriebsstufe 2 | 12,5 bis 11,4°C |
| Abtriebsstufe 3 | 11,2 bis 8,5°C |
| Abtriebsstufe 4 | 8,5 bis 4,3°C |

Bis auf die Abtriebsstufe 4, die statisch gefahren wurde, wurden alle anderen Stufen dynamisch gefahren. Das Verhältnis der in einer Stufe ausgefrorenen Kristallmasse zur der in dieser Stufe eingesetzten Kristallmasse betrug 0,8 in der ersten Reinigungsstufe und 0,75 in der zweiten Reinigungsstufe. In der ersten Reinigungsstufe wurde eine Verunreinigungskonzentration von 0,102 Gew.-% (bezogen auf 100 Gew.-% N-Vinylpyrrolidon) im Kristallisat erreicht. Gegenüber dem US-Patent entspricht dies einer um den Faktor 4 niedrigeren Verunreinigungskonzentration (dort betrug die Verunreinigungskonzentration 0,4 Gew.-% (bezogen auf 100 Gew.-% N-Vinylpyrrolidon)). Das Kristallisat wird danach einer zweiten Reinigungsstufe zugeführt und verläßt diese mit einer Verunreinigungskonzentration von 190 ppm, was, gegenüber dem US-Patent, in dem die Verunreinigungskonzentration 500 ppm betrug, eine etwa um den Faktor 2,5 niedrigere Verunreinigungskonzentration bedeutet.

Im Vergleich zu bekannten Verfahren wird somit mit dem erfindungsgemäßen Verfahren durch die Aufbringung einer Impfschicht aus N-Vinylpyrrolidon vor der jeweiligen Kristallisation bei gleichen Ausgangsbedingungen, gleicher Zahl der Reinigungsstufen und gleicher ausgefrorener Kristallmasse bezogen auf die eingesetzte Schmelze, eine um den Faktor 2,5 niedrigere Verunreinigungskonzentration im gereinigten N-Vinylpyrrolidon erzielt. Bei gleicher Produktspezifikation bedeutet dies eine Einsparung von Reinigungsstufen und damit eine erhebliche Reduzierung des erforderlichen Trennaufwands.

### Beispiel 2

Dieses Beispiel zeigt, daß der Aufwand für die Reinigung von N-Vinylpyrrolidon zusätzlich durch Rückführung des aus der ersten Kristallisationsstufe austretenden Kristallisationsrückstands verringert werden kann, indem hierdurch die Anzahl der Kristallisationsstufen reduziert wird. Ein N-Vinylpyrrolidon-Strom mit 0,8 Gew.-% Verunreinigung (bezogen auf 100 Gew.-% N-Vinylpyrrolidon) wurde einer mehrstufigen Kristallisation unterzogen, wobei gemäß dem erfindungsgemäßen Verfahren in jeder Kristallisationsstufe analog zu Beispiel 1 eine zweiphasige Impfschicht von N-Vinylpyrrolidon auf die Kristallisatorflächen aufgebracht wurde. Die Anzahl der Kristallisationsstufen entsprach der Summe der gemäß untenstehender Tabelle angegebenen Reinigungs- und Abtriebsstufen. Die Gleichgewichtstemperaturen der Schmelze in den einzelnen Stufen waren wie folgt:

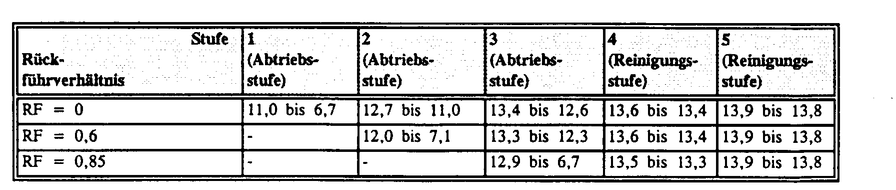

In allen Beispielen wurde in der 2. Reinigungsstufe ein Kristallisatproduktstrom mit < 100 ppm Verunreinigung erhalten. Das Verhältnis von N-Vinylpyrrolidon im Kristallisatproduktstrom in der 2. Reinigungsstufe zu dem (in der 1. Reinigungsstufe) eingesetzten N-Vinylpyrrolidon, d.h., die Ausbeute, betrug mindestens 0,90. Das Verhältnis der in einer Stufe ausgefrorenen Kristallmasse zu der in dieser Stufe eingesetzten Kristallmasse betrug für alle betrachteten Fälle und in allen Stufen 0,65.

Die folgende Tabelle zeigt, daß in Abhängigkeit vom Rückführverhältnis (Rückführung des in der ersten Kristallisationsstufe austretenden Kristallisationsrückstandes in diese Stufe) die Stufenzahl von 5 auf 3 verringert werden kann. Hierbei entspricht die erste Kristallisationsstufe der ersten Abtriebsstufe.

**Tabelle**

| Rückführverhältnis | RF = 0 | RF = 0,6 | RF = 0,85 |
|---|---|---|---|
| Stufenzahl | 5 | 4 | 3 |
| Zahl der Reinigungsstufen | 2 | 2 | 2 |
| Zahl der Abtriebsstufen | 3 | 2 | 1 |
| Verunreinigungskonzentration im Produkt [ppm] | 50 | 51 | 79 |
| Ausbeute | 0,94 | 0,93 | 0,94 |

Wie sich der Tabelle entnehmen läßt, wird durch das erfindungsgemäße Verfahren bei einem Rückführverhältnis von 0,6 oder 0,85 die Zahl der Abtriebsstufen verringert, ohne daß Ausbeuteverluste auftreten oder ein nicht spezifikationsgerechtes Produkt erhalten wird.

Somit bestehen die mit dem erfindungsgemäßen Verfahren erzielbaren Vorteile darin, daß der apparative und energetische Aufwand bei der Reinigung von N-Vinylpyrrolidon erheblich reduziert werden kann. Dies gilt sowohl für die Zahl der zum Erreichen einer bestimmten Produktspezifikation und geforderten Ausbeute erforderlichen Abtriebsstufen als auch Reinigungsstufen des Verfahrens.

## Patentansprüche

1. Verfahren zur Reinigung von N-Vinylpyrrolidon durch Kristallisation in einem Kristallisator, dadurch gekennzeichnet, daß man vor der Kristallisation diejenigen Flächen des Kristallisators, von denen aus während der Kristallisation Kristalle wachsen, mit einer Impfschicht aus N-Vinylpyrrolidon belegt.

2. Verfahren nach Anspruch 1, dadurch gekennzeichnet, daß man die Impfschicht durch Anfrieren eines geschmolzenen Films aus N-Vinylpyrrolidon erzeugt.

3. Verfahren nach Anspruch 1, dadurch gekennzeichnet, daß man die Impfschicht durch Aufbringen einer zweiphasigen Schicht aus N-Vinylpyrrolidon-Schmelze oder -Lösung mit suspendierten N-Vinylpyrrolidon-Kristallen erzeugt.

4. Verfahren nach einem der Ansprüche 1 bis 3, dadurch gekennzeichnet, daß man die Kristallisation in mindestens einer Kristallisationsstufe, davon mindestens einer Reinigungsstufe, insbesondere in drei Kristallisationsstufen, davon in zwei Reinigungsstufen und einer Abtriebsstufe durchführt.

5. Verfahren nach Anspruch 4, dadurch gekennzeichnet, daß man zur Erzeugung der Impfschicht das die jeweilige Kristallisationsstufe verlassende Kristallisat verwendet.

6. Verfahren nach Anspruch 4 oder 5, dadurch gekennzeichnet, daß der in der ersten Kristallisationsstufe austretende Kristallisationsrückstand teilweise dieser Kristallisationsstufe wieder rückgeführt wird.

7. Verfahren nach Anspruch 6, dadurch gekennzeichnet, daß das Verhältnis von austretendem zu rückgeführtem Kristallisationsrückstand (Rückführverhältnis) zwischen 0 und 1,0, bevorzugt zwischen 0,1 und 0,95, liegt.

## Claims

1. A process for purifying N-vinylpyrrolidone by crystallization in a crystallizer, wherein those surfaces of the crystallizer from which crystals grow during the crystallization are covered with an N-vinylpyrrolidone seed layer before the crystallization.

2. A process as claimed in claim 1, wherein the seed layer is produced by freezing a molten film of N-vinylpyrrolidone.

3. A process as claimed in claim 1, wherein the seed layer is produced by applying a two-phase layer of N-vinylpyrrolidone melt or solution with suspended N-vinylpyrrolidone crystals.

4. A process as claimed in any of claims 1 to 3, wherein the crystallization is carried out in at least one crystallization stage, of which at least one is a purification stage, in particular in three crystallization stages, of which two are purification stages and one is a stripping stage.

5. A process as claimed in claim 4, wherein the crystals leaving the particular crystallization stage are used to produce the seed layer.

6. A process as claimed in claim 4 or 5, wherein the crystallization residue emerging from the first crystallization stage is partly returned to this crystallization stage.

7. A process as claimed in claim 6, wherein the ratio of emerging to returned crystallization residue (return ratio) is from 0 to 1.0, preferably from 0.1 to 0.95.

## Revendications

1. Procédé pour la purification de N-vinylpyrrolidone par cristallisation dans un cristallisoir, caractérisé par le fait que, avant la cristallisation, on recouvre avec une couche d'ensemencement de N-vinylpyrrolidone les surfaces du cristallisoir à partir desquelles des cristaux croissent pendant la cristallisation.

2. Procédé selon la revendication 1, caractérisé par le fait qu'on produit la couche d'ensemencement par congélation d'un film fondu de N-vinylpyrrolidone.

3. Procédé selon la revendication 1, caractérisé par le fait que la couche d'ensemencement est produite par dépôt d'une couche biphasée composée d'une masse fondue ou d'une solution de N-vinylpyrrolidone ayant des cristaux de N-vinylpyrrolidone en suspension.

4. Procédé selon l'une des revendications 1 à 3, caractérisé par le fait qu'on réalise la cristallisation en au moins une étape de cristallisation, dont au moins une étape de purification, en particulier en trois étapes de cristallisation, dont deux étapes de purification et une étape d'entraînement.

5. Procédé selon la revendication 4, caractérisé par le fait qu'on emploie pour la production de la couche d'ensemencement le produit de cristallisation provenant à chaque fois de l'étape de cristallisation.

6. Procédé selon la revendication 4 ou 5, caractérisé par le fait que le résidu de cristallisation formé dans la première étape de cristallisation est partiellement recyclé dans cette étape de cristallisation.

7. Procédé selon la revendication 6, caractérisé par le fait que le rapport entre le résidu de cristallisation formé et celui qui est recyclé (rapport de recyclage) se situe entre 0 et 1,0, de préférence entre 0,1 et 0,95.
